# EUROPEAN PATENT SPECIFICATION

(11) **EP 3 150 152 B1**
(45) Date of publication and mention of the grant of the patent: **15.04.2020**
(21) Application number: 15799259.5
(22) Date of filing: 29.05.2015
(51) Int. Cl.: A61B 17/70, A61B 17/90, A61B 34/00

(54) **ASSISTANT ROBOT FOR SPINE SURGERY**
HILFSROBOTER FÜR WIRBELSÄULENCHIRURGIE
ROBOT D'ASSISTANCE POUR CHIRURGIE RACHIDIENNE

(30) Priority: 29.05.2014 KR 20140065284
(43) Date of publication of application: 05.04.2017
(73) Proprietor: Koh Young Technology Inc., Seoul 153-706 (KR)
(72) Inventor: KWON, Young Sik, Ansan-si, Gyeonggi-do 425-875 (KR); HAN, Seung Chul, Asan-si Chungcheongnam-do 336-780 (KR); CHUNG, Jae Heon, Gwangmyeong-si Gyeonggi-do 423-733 (KR)
(74) Representative: Zardi, Marco
(86) International application number: PCT/KR2015/005438
(87) International publication number: WO 2015/183046

(56) References cited:
- WO-A1-2013/018927
- WO-A1-2013/192598
- JP-A- 2006 087 658
- KR-A- 20090 086 656
- KR-A- 20090 119 366
- KR-A- 20110 054 699
- US-B1- 6 699 177
- US-B2- 8 123 675

## Description

### TECHNICAL FIELD

The present disclosure relates to an assistant robot for spine surgery and, more particularly, to an assistant robot for spine surgery which guides the direction of a surgical tool and enables the natural movement and automatic rotation of the surgical tool.

The present disclosure was derived from a research carried out as a part of the industrial source technology development project of the Ministry of Knowledge Economy of the Republic of Korea [Task No.: 10040097, Title: Development of a minimally invasive multi-degree-of-freedom surgical robot system for medical surgery robot image-based otolaryngology neurosurgery operations].

### BACKGROUND ART

The human spine consists of 26 bones, namely 7 cervical vertebrae, 12 thoracic vertebrae, 5 lumbar vertebrae, 1 sacral vertebra and 1 coccygeal vertebra, on an adult basis. 23 intervertebral discs often called discs exist between the respective bones to join and elastically support the bones. Thus, the spine serves to support a human body, maintains equilibrium and protects a spinal cord.

In the spine which is a crucial element that forms a human body, the frequency of occurrence of related diseases is as high as the importance of the spine. As a representative disease causing lumbago, there is a herniation of intervertebral discs. The herniation of intervertebral discs refers to a disease in which an intervertebral disc existing between vertebrae protrudes and stimulates a peripheral nerve, thereby causing pain. The herniation of intervertebral discs is generated by an unstable posture, aging, a strong impact from the outside, or the like.

Typically, when the herniation of intervertebral discs is generated, a conservative treatment method or a surgical treatment method may be used a treatment method thereof. The former is implemented in the case where pain is relatively trivial. Drug therapies and exercises are performed in parallel. If necessary, an assist device may be used. On the other hand, the latter is implemented in the case where a symptom has progressed considerably and the pain is difficult to tolerate. A damaged intervertebral disc is removed and an artificial aid (a cage) or an autogenous bone is inserted in place thereof. Vertebral screws are implanted and fixed to the vertebrae above and below the damaged intervertebral disc. Thereafter, a rod is connected to the vertebral screws to maintain a distance between the vertebrae, thereby ensuring that spinal fusion occurs normally. In addition to the herniation of intervertebral discs, there are many different spine-related diseases such as a spinal stenosis, in which a spinal canal, a neuromuscular canal or an intervertebral foramen becomes narrow, a degenerative spine disease, a vertebral fracture, a spinal anomaly, or the like. The spinal fusion is used for most spinal diseases.

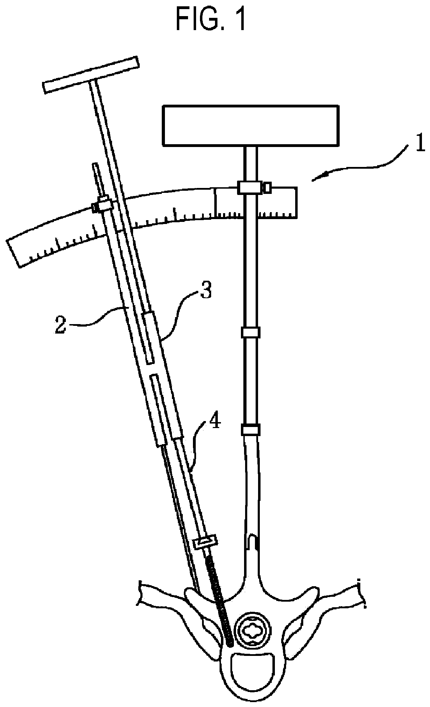

In order to perform surgery such as spinal fusion or the like in which vertebral screws are fixed to vertebrae, it is necessary to use a guide device for accurately maintaining the position of a surgical tool. This kind of guide device for spinal surgery is disclosed in Korean Registered Utility Model Publication No. 20-0198708 Y1 (published on October 2, 2000). **FIG. 1** is a plan view illustrating a guide device for spinal surgery of related art. The guide device includes: a fixing portion 1; a positioning portion 2 fixed to the fixing portion 1; and a guide portion 3 connected to the positioning portion 2. A surgical tool 4 penetrates the guide portion 3. Thus, the guide device serves to restrict the position of the surgical tool 4 while allowing linear motion of the surgical tool 4.

However, the guide device for spinal surgery of related art has no special function other than the function of guiding the direction of the surgical tool 4. Accordingly, when operating the surgical tool 4 as in the case of implanting vertebral screws to vertebrae, an operator as to personally rotate the surgical tool 4. Thus, there is a problem in that it is difficult to accurately and uniformly transmit a rotational force.

In addition, there is a problem in that when the surgical tool 4 is linearly moved, vibration is generated due to the friction between the surgical tool 4 and the guide portion 3.

US 6699177 B1 discloses a system for performing minimally invasive medical procedures including one or more robotic arms that can be attached to the operating table, where the robotic arms can be controlled by input devices such as handles and a foot pedal to perform a minimally medical procedure. WO 2013/018927 A1 discloses an operation support device having a treatment tool unit and a surgical instrument unit support section, the operation support device including a sterilizable intermediate member rotatably connected to the surgical instrument support section and configured to detachably hold the treatment tool unit, a sterilizable drape assembly having hole portions, and a surgical instrument driving unit configured to supply a driving force to the treatment tool unit via the intermediate member. JP 2006 087658 A discloses a frame for a therapeutic apparatus which can change the horizontal position of the therapeutic apparatus without changing its vertical position while avoiding the interference of the frame with the body of a patient.

### SUMMARY

The present disclosure has been made to solve the problems mentioned above. It is an objective of the present disclosure to provide an assistant robot for spine surgery capable of not only guiding the direction of a surgical tool but also enabling the natural movement and automatic rotation of the surgical tool.

According to the present disclosure, there is provided an assistant robot for spine surgery for guiding a surgical tool, comprising: a surgical tool support; a handle; a driving member; and a linear guide, characterized in that the surgical tool support is provided with a rotating body to which the surgical tool is penetratingly coupled; the handle is provided at one side of the surgical tool support; the driving member is configured to rotate the rotating body; and the linear guide is provided at the other side of the surgical tool support so as to move the surgical tool forward and backward.

In the assistant robot for spine surgery according to the present disclosure, the surgical tool can smoothly move without vibration when moving forward and backward and can be automatically rotated with a uniform force so as to enable sophisticated spinal surgery.

### BRIEF DESCRIPTION OF THE DRAWINGS

**FIG. 1** is a plane view showing a guide device for spine surgery of related art.
**FIG. 2** is a perspective view illustrating an assistant robot for spine surgery according to the present disclosure.
**FIG. 3** is an enlarged perspective view of a region indicated by A in **FIG. 2****.**
**FIG. 4** is a side sectional view of a rotating body.
**FIGS. 5A** and **5B** are side views of the rotating body, illustrating a process in which a holder and a holder guide are coupled.
**FIG. 6** is a side view illustrating a state in which the holder and the holder guide are coupled.
**FIG. 7** is an enlarged perspective view of a region indicated by B in **FIG. 2****.**
**FIG. 8** is a perspective view illustrating a state in which the assistant robot for spine surgery according to the present disclosure is applied to a robot arm.

### DETAILED DESCRIPTION

An assistant robot for spine surgery according to the present disclosure will now be described in detail with reference to the accompanying drawings.

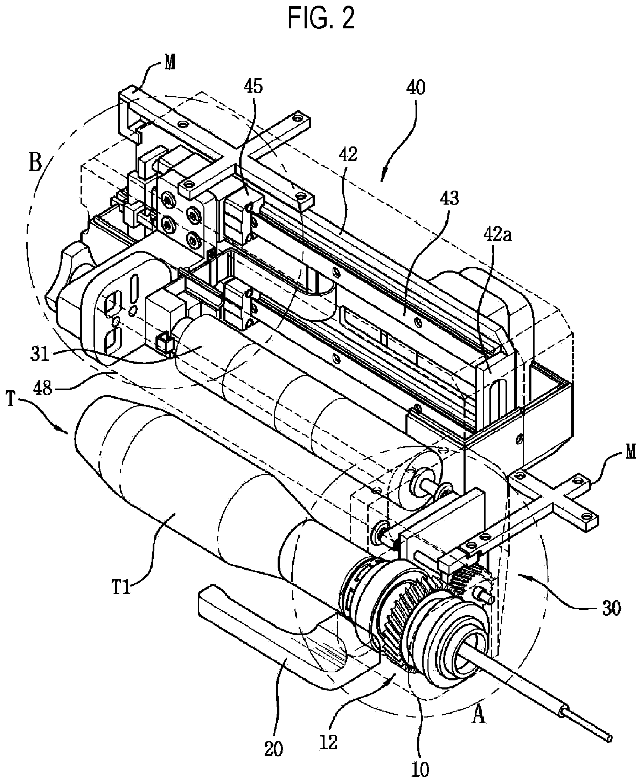

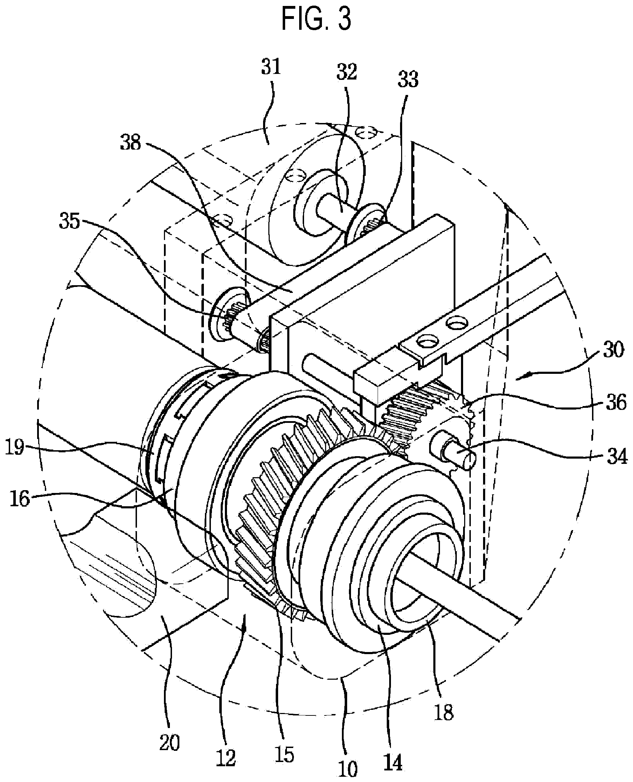

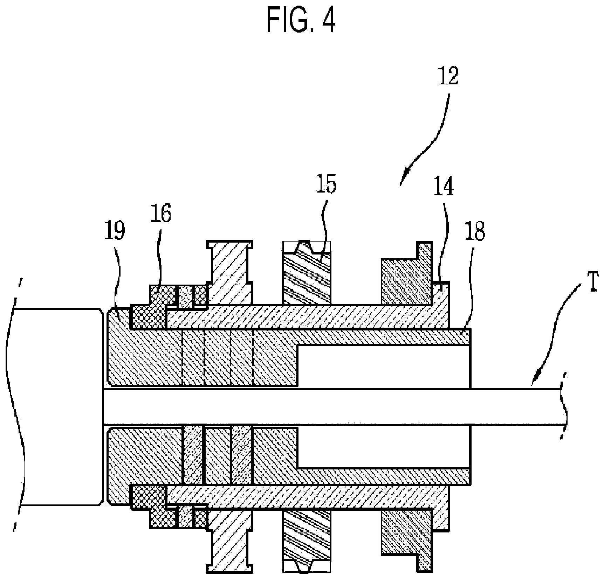

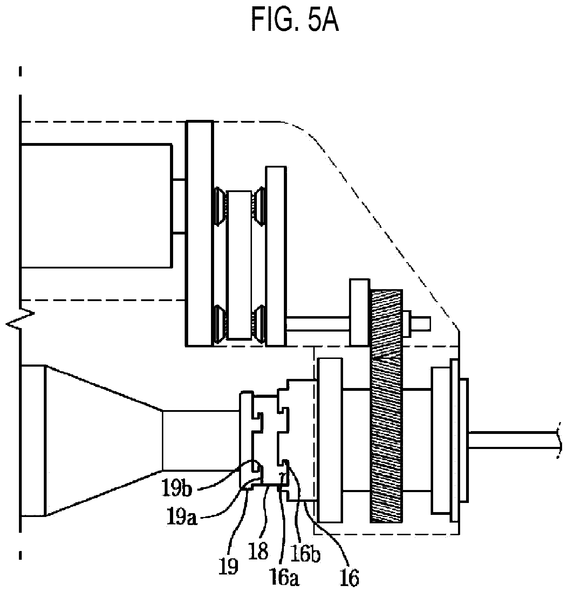

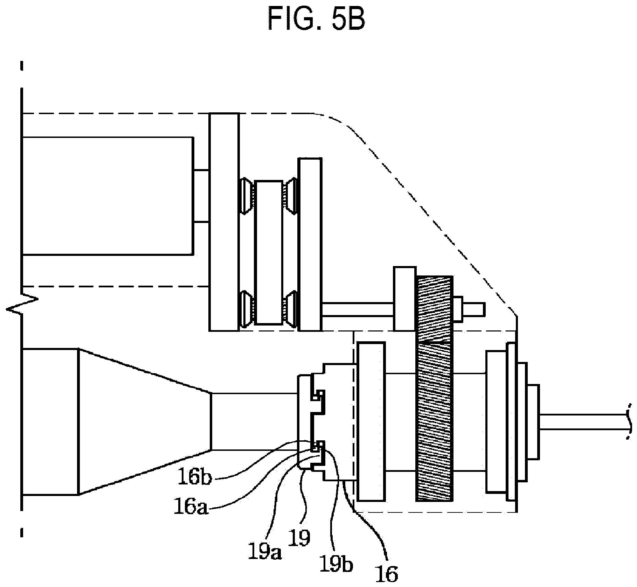

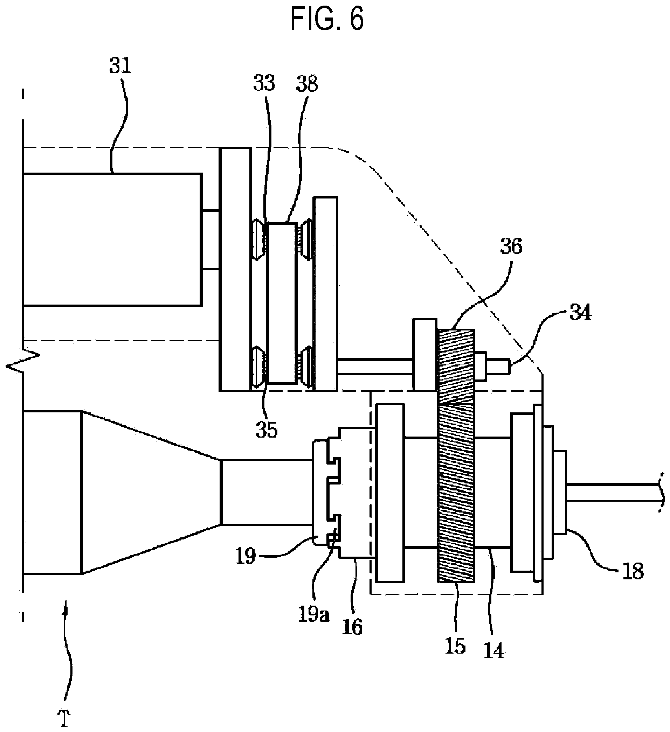

The present disclosure relates to an assistant robot for spine surgery. **FIG. 2** is a perspective view illustrating an assistant robot for spine surgery according to the present disclosure. **FIG. 3** is an enlarged perspective view of a region indicated by A in **FIG. 2****.** **FIG. 4** is a side sectional view of a rotating body. **FIGS. 5A** and **5B** are side views of the rotating body, illustrating a process in which a holder and a holder guide are coupled. **FIG. 6** is a side view illustrating a state in which the holder and the holder guide are coupled.

The assistant robot for spine surgery according to the present disclosure includes: a surgical tool support 10 having a rotating body 12 to which a surgical tool T is penetratingly coupled; a handle 20 provided at one side of the surgical tool support 10; a driving member 30 for rotating the rotating body 12; and a linear guide 40 provided at the other side of the surgical tool support 10 so as to move the surgical tool T forward and backward.

Hereinafter, the respective components will be described in detail.

The surgical tool support 10 is a component for supporting the inserted surgical tool T. The rotating body 12 is positioned within the surgical tool support 10.

The rotating body 12 includes: a first pipe 14 having a driven gear 15 provided on the outer circumferential surface thereof so as to mesh with a driving gear 36 and a holder guide 16 provided at one side thereof; and a second pipe 18 provided with a holder 19 coupled to the holder guide 16, wherein the surgical tool T coupled to the interior of the second pipe 18 and the second pipe 18 configured to rotate together with the first pipe 14. The rotating body 12 is configured to fix the surgical tool T so that the surgical tool T can be rotated.

The first pipe 14 has a hollow pipe shape.

As illustrated in **FIG. 3****,** the driven gear 15 is fixed to the outer circumferential surface of the first pipe 14. Accordingly, if a torque is transmitted from the below-described driving gear 36 to the driven gear 15 and if the driven gear 15 is rotated, the first pipe 14 is rotated together with the driven gear 15.

The holder guide 16 is formed in a ring shape and is fixed to one end side of the first pipe 14. Insertion grooves 16a are formed in the periphery of the holder guide 16.

The second pipe 18 has a hollow pipe shape just like the first pipe 14. As illustrated in **FIG. 4****,** the second pipe 18 is positioned inside the first pipe 14 and is rotated together with the first pipe 14. The surgical tool T is inserted into the second pipe 18 and is firmly fixed to the second pipe 18 by a bolt.

The holder 19 is formed at one end of the second pipe 18, preferably in a portion corresponding to the holder guide 16. The holder 19 is coupled to and meshed with the holder guide 16. For this purpose, coupling projections 19a corresponding to the insertion grooves 16a are formed along the circumferential direction of the holder 19. As a result, the coupling projections 19a of the holder 19 are coupled to the insertion grooves 16a of the holder guide 16, whereby the first pipe 14 is rotated together with the first pipe 14.

If necessary, the surgical tool T may be fixed to the inside of the holder 19 without the second pipe 18.

In the meantime, as illustrated in **FIGS. 5A****,** **5B** and **6****,** the insertion grooves 16a and the coupling projections 19a may be formed in an L-like shape. This is to make sure that the second pipe 18 does not linearly move after the second pipe 18 is coupled to the first pipe 14. Specifically, in the state illustrated in **FIG. 5A****,** if the holder 19 is rotated at a predetermined angle after the coupling projections 19a are inserted into the insertion grooves 16a as illustrated in **FIG. 5B****,** the bent portions 19b of the coupling projections 19a are inserted into the recessed portions 16b of the insertion grooves 16a. Thus, the surgical tool T is not removed backward.

The handle 20 is a component installed at one side of the surgical tool support 10 and used for an operator to arbitrarily move the assistant robot for spine surgery according to the present disclosure. It is preferred that the handle 20 is formed in an outwardly-bent shape in order to broaden the internal space in which the surgical tool T is positioned.

The driving member 30 is a component connected to the surgical tool support 10 and configured to rotate the rotating body 12. The driving member 30 includes: a motor 31; and a driving gear 36 connected to the motor 31.

The motor 31 is a driving power source connected to the surgical tool support 10 and configured to supply a torque to the surgical tool T. If necessary, the motor 31 may be installed within the handle 20 or may be installed within a below-described guide bar 48, thereby enhancing the space use efficiency.

The driving gear 36 is meshed with the driven gear 15 of the surgical tool support 10 and is configured to transmit the torque of the motor 31 to the surgical tool T.

In the meantime, the assistant robot for spine surgery according to the present disclosure may further include a speed reducer disposed between the motor 31 and the driving gear 36 to accurately adjust the rotation of surgical tool T. The speed reducer includes: a first shaft 32 connected to the motor 31 and provided with a first gear 33; a second shaft 34 provided with a second gear 35 corresponding to the first gear 33 and the driving gear 36, and disposed parallel to the first shaft 32; and a belt 38 configured to interconnect the first gear 33 and the second gear 35.

The first shaft 32 is connected to the motor 31 and is rotated in response to the operation of the motor 31. Further, the first gear 33 is fixed to the first shaft 32 and is rotated together with the first shaft 32.

As illustrated in **FIG. 3****,** the second shaft 34 is disposed parallel to the first shaft 32. The second gear 35 is fixed and installed to the position facing with the first gear 33 of the second shaft 34. The belt 38 is connected to the first gear 33 and the second gear 35 so that the torque of the first gear 33 is transmitted to the second gear 35. In this regard, the belt 38 serves to efficiently increase the distance between the first shaft 32 and the second shaft 34. Specifically, in order to achieve such an effect without the belt 38, it is necessary to further install an intermediate gear. There is a problem in that this structure becomes complex and the power loss occurs. The present disclosure minimizes the aforementioned problem by installing the belt 38 between the first gear 33 and the second gear 35.

If necessary, teeth such as racks or the like may be formed on the inner surface of the belt 38, or an idler (not shown) may be installed midway of the belt 38, thereby increasing the contact force of the belt 38 with the gears.

The driving gear 36 is installed in the second shaft 34 in a position where the driving gear 36 meshes with the driven gear 15. The driving gear 36 is configured to transmit the torque of the motor 31 to the driven gear 15. The driven gear 15 and the driving gear 36 may be formed of helical gears. This makes it possible to minimize a gear friction noise otherwise generated during surgery and to enable sophisticated control.

On the other hand, in the assistant robot for spine surgery according to the present disclosure, the surgical tool T may be automatically rotated by the driving member 30. However, if necessary, an operator may manually rotate the surgical tool T using a handle T1 of the surgical tool T.

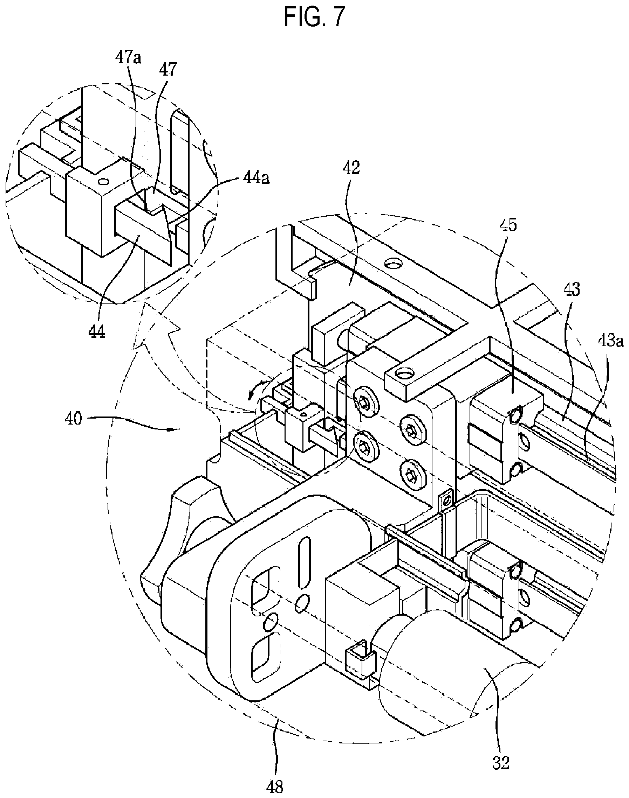

**FIG. 7** is an enlarged perspective view of a region indicated by B in **FIG. 2****.**

The linear guide 40 is a component installed in the surgical tool support 10 to provide one degree of freedom to the surgical tool support 10. Specifically, the linear guide 40 is configured to move the surgical tool support 10 forward and backward.

The linear guide 40 includes: a base plate 42 provided with a guide rail 43 formed on an upper surface thereof; a sliding block 45 coupled to the guide rail 43 so as to slide along the guide rail 43; and a guide bar 48 coupled to the sliding block 45 and configured to support the surgical tool support 10.

The base plate 42 is a stand that forms a base of the linear guide 40. The base plate 42 includes a front end portion 42a which protrudes upward so that the below-described sliding block 45 is not disassembled.

The guide rail 43 is installed on the upper surface of the base plate 42 and is formed to extend along the longitudinal direction of the base plate 42. From the viewpoint of stability, it is advantageous to be provided a pair of guide rails rather than the single guide rail 43. Further, grooves 43a are formed on the opposite side surfaces of the guide rail 43.

A hook 44 is installed at the rear side of the upper portion of the base plate 42. Referring to **FIG. 7****,** the hook 44 is coupled to the base plate 42 by a shaft in the same manner as a lever. An elastic member such as a torsion spring or the like is coupled to the shaft, and thereby the hook 44 may be elastically operated. The hook 44 includes an inclined surface 44a formed at the tip thereof.

The sliding block 45 is formed in conformity with the shape of the grooves 43a of the guide rail 43. The sliding block 45 is coupled to the guide rail 43 and is linearly moved along the guide rail 43.

An engagement projection 47 having an inclined surface 47a formed at the tip thereof so as to correspond to the shape of the hook 44 is formed at the rear side of the sliding block 45. Thus, if the sliding block 45 reaches the rear end of the guide rail 43 while the sliding block 45 moves forward and backward along the guide rail 43, the inclined surface 47a of the engagement projection 47 makes contact with the inclined surface 44a of the hook 44 and lifts up the hook 44. Thereafter, the engagement projection 47 is brought into engagement with the hook 44. Accordingly, the assistant robot for spine surgery according to the present disclosure can prevent the surgical tool support 10, the handle 20 and the driving member 30 from being arbitrarily moved along the guide rail 43 by their own weights.

The guide bar 48 is connected at one end to the sliding block 45 and at the other end to the surgical tool support 10 so that the surgical tool support 10 and the sliding block 45 move together. In one embodiment of the present disclosure, the driving member 30 may be installed within the guide bar 48.

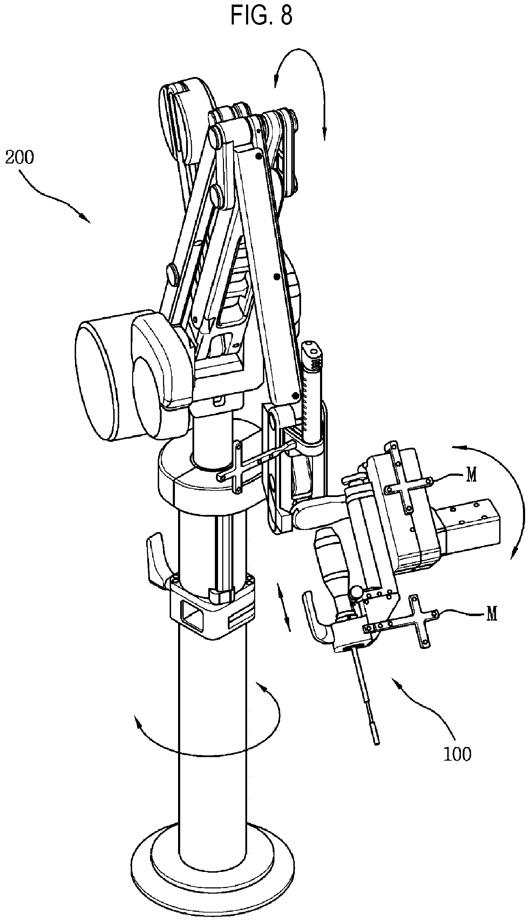

**FIG. 8** is a perspective view illustrating a state in which the assistant robot for spine surgery according to the present disclosure is applied to a robot arm.

As illustrated in **FIG. 8****,** the assistant robot for spine surgery 100 according to the present disclosure may be mounted to a robot arm 200 having multiple degree of freedom and may be moved to various positions. And a marker M is installed so as to trace the position of the assistant robot for spine surgery 100.

### (EXPLANATION OF REFERENCE NUMERAL)

T: surgical tool, T1: surgical tool handle
10: surgical tool support, 12: rotating body
14: first pipe, 15: driven gear
16: holder guide, 16a: insertion grooves
18: second pipe, 19: holder
19a: coupling projections, 20: handle
30: driving member, 31: motor
32: first shaft, 33: first gear
34: second shaft, 35: second gear
36: driving gear, 38: belt
40: linear guide, 42: base plate
43: guide rail, 44: hook
44a & 47a: inclined surface, 45: sliding block
47: engagement projection, 48: guide bar
100: assistant robot for spine surgery, 200: robot arm

## Claims

1. An assistant robot for spine surgery for guiding a surgical tool (T), comprising a surgical tool support (10); a driving member (30); and a linear guide (40),
the surgical tool support (10) being provided with a rotating body (12) to which the surgical tool (T) is penetratingly coupled;
the driving member (30) being configured to rotate the rotating body (12); and
the linear guide (40) being provided at the other side of the surgical tool support (10) so as to move the surgical tool (T) forward and backward,
**characterised by** a handle (20), wherein the handle (20) is provided at one side of the surgical tool support (10).

2. The assistant robot of Claim 1, wherein the driving member (30) includes a motor (31) and a driving gear (36) connected to the motor (30), and
the rotating body (12) includes a first pipe (14) having a driven gear (15) provided on an outer circumferential surface of the first pipe (14) so as to mesh with the driving gear (36) and a holder guide (16) provided at one side of the first pipe (14), and a second pipe (18) provided with a holder (19) coupled to the holder guide (16), the surgical tool (T) coupled to an interior of the second pipe (18), the second pipe (18) configured to rotate together with the first pipe (14).

3. The assistant robot of Claim 2, wherein the holder guide (16) is formed in a ring shape and provided with insertion grooves (16a) formed in a periphery of the holder guide (16), and the holder (19) is formed in a ring shape and provided with coupling projections (19a) inserted into the insertion grooves (16a).

4. The assistant robot of Claim 3, wherein the coupling projections (19a) and the insertion grooves (16a) are formed in an L-like shape.

5. The assistant robot of Claim 2, wherein the driving member (30) includes a first shaft (32) connected to the motor (30) and provided with a first gear (33), a second shaft (34) provided with a second gear (35) corresponding to the first gear (33) and the driving gear (30) and disposed parallel to the first shaft (32), and a belt (38) configured to interconnect the first gear (33) and the second gear (35).

6. The assistant robot of Claim 1, wherein the linear guide (40) includes a base plate (42) provided with a guide rail (43) formed on an upper surface of the base plate (42), a sliding block (45) coupled to the guide rail (43) so as to slide along the guide rail (43), and a guide bar (48) coupled to the sliding block (45) and configured to support the surgical tool support (10).

7. The assistant robot of Claim 6, wherein the sliding block (45) is provided with an engagement projection (47), and the base plate (42) is provided with a hook (44) elastically coupled to the engagement projection (47).

## Patentansprüche

1. Assistenzroboter für Wirbelsäulenchirurgie zum Führen eines chirurgischen Werkzeugs (T), umfassend einen Chirurgiewerkzeughalter (10); ein Antriebselement (30) und eine Linearführung (40),
wobei der Chirurgiewerkzeughalter (10) mit einem rotierenden Körper (12) versehen ist, mit dem das chirurgische Werkzeug (T) in durchdringender Weise gekoppelt ist;
wobei das Antriebselement (30) dazu konfiguriert ist, den rotierenden Körper (12) zu drehen; und
die Linearführung (40) an dem anderen Ende des Chirurgiewerkzeughalters (10) vorgesehen ist, um das chirurgische Werkzeug (T) vorwärts und rückwärts zu bewegen,
**gekennzeichnet durch**
einen Griff (20), wobei der Griff (20) an einer Seite des Chirurgiewerkzeughalters (10) vorgesehen ist.

2. Assistenzroboter nach Anspruch 1, wobei das Antriebselement (30) einen Motor (31) und ein mit dem Motor (30) verbundenes Antriebszahnrad (36) umfasst; und
der rotierende Körper (12) ein erstes Rohr (14), das ein angetriebenes Zahnrad (15), das auf einer Außenumfangsoberfläche des ersten Rohrs (14) vorgesehen ist, um mit dem Antriebszahnrad (36) in Eingriff zu stehen, und eine Halterführung (16) aufweist, die an einer Seite des ersten Rohrs (14) vorgesehen ist, und ein zweites Rohr (18) umfasst, das mit einem Halter (19) versehen ist, der mit der Halterführung (16) gekoppelt ist, wobei das chirurgische Werkzeug (T) mit einem Inneren des zweiten Rohrs (18) gekoppelt ist, und das zweite Rohr (18) dazu konfiguriert ist, sich zusammen mit dem ersten Rohr (14) zu drehen.

3. Assistenzroboter nach Anspruch 2, wobei die Halterführung (16) in einer Ringform gebildet und mit Einfügenuten (16a) versehen ist, die in einem Umfang der Halterführung (16) gebildet sind, und der Halter (19) in einer Ringform gebildet und mit Kopplungsvorsprüngen (19a) versehen ist, die in die Einfügenuten (16a) eingefügt sind.

4. Assistenzroboter nach Anspruch 3, wobei die Kopplungsvorsprünge (19a) und die Einfügenuten (16a) in einer L-artigen Form gebildet sind.

5. Assistenzroboter nach Anspruch 2, wobei das Antriebselement (30) eine erste Welle (32), die mit dem Motor (30) verbunden und mit einem ersten Zahnrad (33) versehen ist, eine zweite Welle (34), die mit einem dem ersten Zahnrad (33) entsprechenden zweiten Zahnrad (35) und mit dem Antriebszahnrad (30) versehen und parallel zu der ersten Welle (32) angeordnet ist, und einen Riemen (38) umfasst, der dazu konfiguriert ist, das erste Zahnrad (33) und das zweite Zahnrad (35) miteinander zu verbinden.

6. Assistenzroboter nach Anspruch 1, wobei die Linearführung (40) eine Basisplatte (42), die mit einer auf einer oberen Oberfläche der Basisplatte (42) gebildeten Führungsschiene (43) versehen ist, einen Gleitblock (45), der mit der Führungsschiene (43) gekoppelt ist, um entlang der Führungsschiene (43) zu gleiten, und eine Führungsstange (48) umfasst, die mit dem Gleitblock (45) verbunden und dazu konfiguriert ist, den Chirurgiewerkzeughalter (10) zu tragen.

7. Assistenzroboter nach Anspruch 6, wobei der Gleitblock (45) mit einem Eingriffsvorsprung (47) versehen ist, und die Basisplatte (42) mit einem Haken (44) versehen ist, der mit dem Eingriffsvorsprung (47) elastisch gekoppelt ist.

## Revendications

1. Robot d'assistance pour chirurgie de la moelle épinière pour guider un outil chirurgical (T), comprenant un support pour outil chirurgical (10), un élément d'entraînement (30) et un guide linéaire (40),
le support pour outil chirurgical (10) étant pourvu d'un corps pivotant (12) auquel l'outil chirurgical (T) est associé par insertion,
l'élément d'entraînement (30) étant configuré pour faire pivoter le corps pivotant (12) et
le guide linéaire (40) étant prévu au niveau de l'autre face du support pour outil chirurgical (10) de manière à mouvoir l'outil chirurgical (T) vers l'avant et l'arrière, **caractérisé par** une poignée (20), où la poignée (20) est prévue au niveau d'un côté du support pour outil chirurgical (10) .

2. Robot d'assistance selon la revendication 1, **caractérisé en ce que** l'élément d'entraînement (30) inclut un moteur (31) et un engrenage d'entraînement (36) connecté au moteur (30) et
le corps pivotant (12) inclut un premier tube (14) présentant un engrenage d'entraînement (15) prévu sur une surface de circonférence externe du premier tube (14) de manière à interagir avec l'engrenage d'entraînement (36) et un guide du mainteneur (16) prévu au niveau d'un côté du premier tube (14) et un deuxième tube (18) pourvu d'un mainteneur (19) couplé au guide du mainteneur (16), l'outil chirurgical (T) couplé à un intérieur du deuxième tube (18), le deuxième tube (18) configuré pour pivoter ensemble avec le premier tube (14).

3. Robot d'assistance selon la revendication 2, **caractérisé en ce que** le guide du mainteneur (16) est façonné en forme d'anneau et pourvu de gorges d'insertion (16a) façonnées dans une périphérie du guide du mainteneur (16) et le mainteneur (19) est façonné en forme d'anneau et pourvu de protubérances de couplage (19a) insérées dans les gorges d'insertion (16a).

4. Robot d'assistance selon la revendication 3, **caractérisé en ce que** les protubérances de couplage (19a) et les gorges d'insertion (16a) sont façonnées en forme de L.

5. Robot d'assistance selon la revendication 2, **caractérisé en ce que** l'élément d'entraînement (30) inclut un premier arbre (32) connecté au moteur (30) et pourvu d'un premier engrenage (33), un deuxième arbre (34) pourvu d'un deuxième engrenage (35), correspondant au premier engrenage (33) et à l'engrenage d'entraînement (30), et placé parallèle au premier arbre (32) et une courroie (38) configurée pour interconnecter le premier engrenage (33) et le deuxième engrenage (35).

6. Robot d'assistance selon la revendication 1, **caractérisé en ce que** le guide linéaire (40) inclut une plaque de base (42) pourvue d'un rail de guidage (43) formé sur une surface supérieure de la plaque de base (42), un bloc coulissant (45) couplé au rail de guidage (43) de façon à coulisser le long du rail de guidage (43) et une barre de guidage (48) couplée au bloc coulissant (45) et configurée pour porter le support pour outil chirurgical (10).

7. Robot d'assistance selon la revendication 6, **caractérisé en ce que** le bloc coulissant (45) est pourvu d'une protubérance d'engagement (47) et **en ce que** la plaque de base (42) est pourvue d'un crochet (44) couplé de manière élastique à la protubérance d'engagement (47).
